Europäisches Patentamt

European Patent Office

Office européen des brevets

(19)

(11) Veröffentlichungsnummer: **0 001 099**
**B1**

(12) # EUROPÄISCHE PATENTSCHRIFT

(21) Anmeldenummer: **78100803.2**

(22) Anmeldetag: **01.09.78**

(51) Int. Cl.³: **C 12 P 19/12, C 07 H 3/04// A 23 K 1/00**

(54) Verfahren zur kontinuierlichen Isomerisierung von Saccharose zu Isomaltulose mit Hilfe von Mikroorganismen

(30) Priorität: **13.09.77 DE 2741197**
**14.02.78 DE 2806216**

(43) Veröffentlichungstag der Anmeldung:
**21.03.79 Patentblatt 79/06**

(45) Bekanntmachung des Hinweises auf die Patenterteilung:
**20.08.80 Patentblatt 80/17**

(84) Benannte Vertragsstaaten:
**BE CH DE FR GB NL SE**

(56) Entgegenhaltungen:
**Chemical Abstracts, Vol. 53, 1959, 3745h Silvia Lorenz "Bacteriological process control, and paperchromatographic studies of sucrose conversion by bacteria"**
**Chemical Abstracts, Vol. 55, 1961, 27517g, E. J. Bourné "Oligosaccharides in dextran-producing cultures of streptococcus bovis"**

(73) Patentinhaber: **BAYER AG**
**Zentralbereich Patente, Marken und Lizenzen**
**D - 5090 Leverkusen 1, Bayerwerk (DE)**

(72) Erfinder: **Crueger, Wulf, Dr.**
**Ruhrstrasse 35**
**D - 4006 Erkrath 2 (DE)**
**Drath, Lore, Dr.**
**Siegfriedstrasse 20**
**D - 6520 Worms (DE)**
**Munir, Mohammed, Dr.**
**Wormser Strasse 1**
**D - 6719 Obrigheim (DE)**

Verfahren zur kontinuierlichen Isomerisierung von Saccharose zu Isomaltulose mit Hilfe von Mikroorganismen

Gegenstand der Erfindung ist ein Verfahren zur kontinuierlichen Isomerisierung von Saccharose zu Isomaltulose (Palatinose, $\alpha$-D-Glucopyranosido-1,6-fructose) mit Hilfe von Mikroorganismen.

Isomaltulose ist ein Zwischenprodukt zur Herstellung von Glucopyranosido-1,6-mannit (DT-OS 2 520 173) und Glucopyranosido-1,6-sorbit (Isomaltit, DT-PS 2 217 628). Beide Substanzen können als Zuckeraustauschstoffe eingesetzt werden.

Nach der deutschen Patentschrift 1 049 800 ist bekannt, daß Saccharose enzymatisch in Isomaltulose umgewandelt wird. Die Enzyme dazu sind mikrobiellen Ursprungs. Neben Protaminobacter rubrum sind weitere Bakterien wie Erwinia carotovora, Serratia marcescens, Serratia plymuthica und Leuconostoc mesentheroides zu dieser Umlagerung befähigt (S. Schmidt-Berg-Lorenz, W. Mauch, Zeitschrift für die Zuckerindustrie *14*, 625—627 (1964); F. H. Stodola 126th Meeting Amer. Chem. Soc., Sept. 1954, Abstracts of Papers, S. 5 D; W. Mauch, S. Schmidt-Berg-Lorenz, Zeitschrift f. d. Zuckerindustrie *14*, 309—315 und 375—383 (1964)).

Weiterhin ist nach der deutschen Patentschrift 2 217 628 bekannt, daß die enzymatische Umwandlung von Saccharose in Isomaltulose mit einer 15—40 %igen Lösung unter starkem Rühren und aeroben Bedingungen bei 20—37°C chargenweise oder kontinuierlich durchgeführt werden kann. Nach Beendigung der enzymatischen Reaktion wird nach obiger Patentschrift die Bakteriensuspension über einen Separator abgetrennt und bis zu sechsmal wieder verwendet. Bei der kontinuierlichen Umsetzung der Saccharose werden nach der gleichen Patentschrift die Bakterien auf einer speziellen Nährlösung angezüchtet und kontinuierlich abgezogen. Die Umsetzung erfolgt dann in einem zweiten Kesselkadensystem, in dem Bakterienkultur und Dicksaft aus der Zuckerfabrikation oder eine andere zuckerhaltige Lösung gehalten werden.

Die getrennte Züchtung der Mikroorganismen von der enzymatischen Umsetzung der Saccharose ist aber aus verschiedenen Grüden unbefriedigend:

a) Die Mikroorganismen werden in einem eigenen Medium angezogen, das erhöhte Kosten verursacht.

b) Die Kulturlösung und auch der Fermenter müssen unabhängig vom Reaktorkesselsystem, in dem die enzymatische Umlagerung erfolgt, sterilisiert und steril gehalten werden.

c) Die Züchtung der Mikroorganismen in einer Nährlösung, die unterschiedlich von der späteren Umsatzlösung ist, birgt die Gefahr, daß die Mikroorganismen während der Fermentation nach anderen Kriterien als dem der maximalen Umsatzrate selektioniert werden.

d) Das Abtrennen der Mikroorganismen und die Wiederverwendung für weitere enzymatische Umsetzungen ist im technischen Maßstab mit einer hohen Infektionsgefahr verbunden.

Es wurde nun gefunden, daß Saccharose kontinuierlich zu Isomaltulose isomerisiert werden kann, wenn man isomaltulosebildende Mikroorganismen in einem saccharosehaltige Pflanzensäfte enthaltenden Nährmedium in an sich bekannter Weise bebrütet, nach Erreichen einer ausreichenden Zelldichte kontinuierlich weiterzüchtet, indemman mit einer Verdünnungsrate, die zwischen 0,05 und 0,3 pro Stunde liegt, kontinuierlich saccharosehaltige Lösungen zusetzt, nach Isomerisierung der Saccharose die Lösung von der Mikroorganismenmasse abtrennt und in an sich bekannter Weise reinigt und/oder zur Kristallisation bringt.

Unter isomaltulosebildenden Mikroorganismen wurden hier vor allem Protaminobacter rubrum, außerdem Serratia plymuthica, Serratia marcescens, Erwinia carotovora und Leuconostoc mesentheroides verstanden.

Es war überraschend und nicht zu erwarten daß isomaltulosebildende Mikroorganismen, z.B. Protaminobacter rubrum, (CBS 574.77) in saccharosehaltigen Pflanzensäften, die z.B. bei der Rohr- und Rübenzuckerherstellung als Zwischenprodukt anfallen und damit billiger als reine Saccharoselösung sind, während des Wachstums gleichzeitig Saccharose in Isomaltulose überführen. Dabei ist gemäß der Erfindung das Wachstum der Organismen mit der Umsetzungsrate so gekoppelt, daß der gesamte Prozeß, d.h. Wachstum und Umsetzung, nur über einen kritischen Parameter, wie z.B. $CO_2$-Gehalt in der Abluft, Sauerstoffpartialdruck in der Kulturlösung oder aber über die Umsetzungsrate gesteuert werden kann.

Ein weiterer Vorteil des Verfahrens ist der folgende: Während der Umlagerung zu Isomaltulose entstehen geringe Mengen Fructose und Glukose, die den Mikrooganismen als Kohlenhydrate für ihren Stoffwechsel dienen. Es müssen nach dem Verfahren keine weiteren Kohlenhydratquellen für das Wachstum zugesetzt werden. Gleichzeitig wird ein weitgehender Abbau dieser unerwünschten Nebenprodukte erzielt.

Bei der kontinuierlichen Isomaltulose-Fermentation verwendet man als Ausgangslösung Dünnsaft/Dicksaft- und/oder Dünnsaft/Kläre-Gemische aus einer Zuckerfabrik mit einem Trockensubstanzgehalt von 5—30%, vorzugsweise von 20—27%. Der Saccharosegehalt, bezogen auf Trockengewicht, beträgt 90—98%, vorzugsweise 94—96%.

Um ein optimales Wachstum der Bakterienkultur zu erlangen, werden Phosphat-Ionen in Form von Salzen wie z.B. $(NH_4)_2HPO_4$ oder

$K_2HPO_4$ in Konzentrationen von 0,05—1 g/l, vorzugsweise von 0,1—0,5 g/l, zugegeben. Normalerweise enthalten die Zwischenprodukte der Zuckerfabrikation nach der Saftreinigung noch einen Rest-CaO-Gehalt von 40—150 mg auf 100 g TS. Durch die Zugabe von $PO_4^{3-}$ wird dieser Rest-CaO-Gehalt als Calcium-phosphat gefällt und somit geht ein Teil des zugesetzten Phosphates für die Zellvermehrung verloren.

Daher wird vorteilhafterweise die zur Fermentation gelangende Zuckerlösung über einen Basenaustauscher enthärtet, wodurch der Zusatz an $PO_4^{3-}$-Ionen gesenkt werden kann.

Die Nährlösung wurde batchweise oder kontinuierlich sterilisiert; eine pH-Korrektur ist nicht nötig, wenn der pH-Wert der verwendeten Zuckerlösung zwischen 7,5 und 8,5 liegt.

Der Fermenter wird mit 0,1—10% Impfmaterial, angezogen in der gleichen Nährlösung mit Erlenmeyer-Kolben auf der Schüttelmaschine, beimpft und bei einer Temperatur von 18—32°C, vorzugsweise bei 30°C, mit einer Belüftungsrate von 0,2—1,0 Volumen Luft pro Volumen Nährlösung mal Minute (vvm), vorzugsweise 0,4 vvm, bei ausreichender Rührung bebrütet.

Nach Erreichen einer ausreichenden Zelldichte, z.B. $10^9$ Keime pro ml wird die Fermentation kontinuierlich durchgeführt, d.h. pro Zeiteinheit werden gleiche Anteile der Kulturlösung entnommen und frische, sterile, nicht beimpfte Nährlösung aus einem Vorratstank zugefügt. Bei einer optimalen Verdünnungsrate, die zwischen 0,05 und 0,3 Stunden$^{-1}$, vorzugsweise bei 0,2 Stunden$^{-1}$ liegt kommt es nicht zu einem Auswaschen der Bakterien. Die Bakterien wachsen im "Steady state" so schnell, daß eine 90—100%ige Umsetzung in Isomaltulose erzielt wird. Eine Stabilisierung des pH-Wertes während der Umsetzung ist in der Regel nicht erforderlich.

Die Verdünnungsrate und damit der Abfluß der Kulturlösung aus der kontinuierlichen Fermentation wird unter anderem mit sterilisierbaren Sauerstoffelektroden über den Sauerstoffpartialdruck in der Kulturlösung oder über den $CO_2$-Gehalt in der Abluft oder über weitere physiologische Parameter gesteuert. Eine weitere Möglichkeit der Steuerung der kontinuierlichen Fermentation ist die Kontrolle der Umsatzrate von Saccharose in Isomaltulose durch Bestimmung des Reduktionsvermögens der Fermentationslösung. (Bekanntlich beträgt das Reduktionsvermögen der Isomaltulose 52% dessen der Glukose, während Saccharose keine reduzierenden Eigenschaften besitzt.) Eine zweite Möglichkeit der Kontrolle der Umsetzungsrate ist der quantitative Nachweis von Isomaltulose neben Saccharose mit Hilfe der Hochdruckflüssigkeitschromatographie.

Die aus dem Fermenter abgezogene Nährlösung wird anschließend von den Mikroorganismen z.B. mit einem Separator getrennt und weiter aufgearbeitet. Die abgeschleuderte Bakterienmasse wird ein zweitesmal mit Wasser aufgeschwemmt, zentrifugiert und kann unter anderem als Viehfutter verwendet werden.

Falls die aus dem Fermenter abgezogene Kulturlösung nur z.B. zu 90% umgesetzt ist, wird durch die Mikroorganismen ohne erneute Zufuhr von Sauerstoff im Leitungssystem bis zum Separator die vollständige Umsetzung durchgeführt.

### Beispiel 1

a) Von einer Abimpfung des Stammes Protaminobacter rubrum Z 12 (CBS 574.77) werden Zellen mit 10 ml einer sterilen Mischung von einem Teil Dicksaft (TS = 65%) und zwei Teilen Leitungswasser plus 0,5 g/l $(NH_4)_2HPO_4$ (falls erforderlich mit HCl auf pH 7,2 eingestellt) abgeschwemmt. Diese Suspension dient als Impfgut für die Schüttelmaschinen-Vorkultur in 1 l - Kolben mit 200 ml Nährlösung obiger Zusammensetzung (Sterilisation 20 min. bei 121°C). Nach einer 30-stündigen Bebrütungszeit bei 29°C werden mit je 20 Kolben (4 l) 16 l Nährlösung obiger Zusammensetzung in einem 30-l-Klein-fermenter beimpft und bei 18°C mit 20 l Luft pro Minute und einer Rührgeschwindigkeit von 350 Upm fermentiert. Die Umsetzung der Saccharose in Isomaltulose wird durch die quantitative Bestimmung des Reduktionsverhaltens der Nährlösung mit Hilfe von Müller'sche Lösung (Zeitschrift Wirt. Zuckerind. Techn. T. *86*, S. 130 und S. 322 (1936) und Zeitschrift Wirt. Zuckerind. Techn. T. *88*, S. 280 (1938) verfolgt. Die Keimzahl wird mikrobiologisch bestimmt. Nach Erreichen von $1 . 10^9$ Keimen/ml wird mit einer stündlichen Verdünnungsrate von 0,09 h$^{-1}$ eine 100%ige Umsetzung erzielt.

b) Impfgut und Vorkultur vom Stamm Protaminobacter rubrum werden entsprechend Beispiel a) bis zum 30-l-Maßstab hergestellt, jedoch jeweils bei Temperaturen von 29°C. 20 l angewachsende Nährlösung aus dem Klein-fermenter dienen als Impfmaterial für einen 300-l-Fermenter mit 180 l Nährlösung (Dicksaft - Saccharose - Wasser - Gemisch mit 25% Trockensubstanzgehalt und einer Reinheit von 96%, bezogen auf TS). Der Fermenter läuft mit einer Belüftungsrate von 200 l Luft/min., einer Temperatur von 29°C und einer Rührgeschwindigkeit von 200 Upm.

Nach einer Anwachsphase von 7 Stunden wird mit einer Verdünnungsrate von 0,09h$^{-1}$ eine 100%ige Umsatzrate erzielt.

c) Impfgut und Vorkultur vom Stamm Protaminobacter rubrum werden entsprechend Beispiel a) bis zur Schüttelkultur hergestellt. Mit 2 l Impfgut von der Schüttelmaschine (=1%) wird ein Fermenter entsprechend b) angeimpft, Fermentationsbedingungen entsprechend Beispiel b). Nach der Anwachsphase von 15 Stunden wird mit einer Verdünnungsrate von 0,2 h$^{-1}$ eine 100%ige Umsatzrate erzielt.

d) Impfgut und Vorkultur vom Stamm Protaminobacter rubrum werden entsprechend Beispiel a) bis zur Schüttelkultur hergestellt. Mit

0,2 l Impfgut wird ein Fermenter entsprechend Beispiel b) angeimpft (0,1%), Fermentationsbedingungen entsprechend Beispiel b). Nach einer Anwachsphase von 23 Stunden wird mit einer Verdünnungsrate von 0,25 h$^{-1}$ ein 90%iger Umsatz erzielt. Beim anschließenden Transport der abgezogenen Nährlösung zum Separator setzt sich ohne zusätzliche Belüftung die Umsetzung fort, so daß in den von den Bakterien getrennten Nährlösungen keine Saccharose mit Hilfe der Flüssigkeitshochdruckchromatographie mehr nachweisbar ist.

e) Ein 3000-l-Fermenter wird mit 1800 l Nährlösung (Dicksaft - Saccharose - Wasser - Gemisch, 25% Trockensubstanzgehalt, Reinheit 98%) und 200 l Impfgut aus einer weiteren 2000-l-Fermentation gefahren. Die Temperatur beträgt 30°C. Die Belüftungsrate 0,4 vvm. die Rührgeschwindigkeit 140 Upm. Die Fermentation wird über den $CO_2$-Gehalt in der Abluft verfolgt. Nach Erreichen von 2,6% $CO_2$ in der Abluft wird mit einer Verdünnungsrate von 0,13 h$^{-1}$ gefahren, wobei in der abfließenden Kulturlösung keine Saccharose, sondern nur noch Isomaltulose nachgewiesen wird.

### Beispiel 2

a) Von einer Abimpfung des Stammes Serratia plymuthica (ATCC 15 928) werden Zellen mit 10 ml einer Dicksaftlösung (25% TS-Gehalt, 96% Reinheit, 0,5 g/l $(NH_4)_2HPO_4$-Zusatz) abgeschwemmt. Diese Suspension dient als Impfgut für eine Schüttelmaschinen-Vorkultur in einem 1-l-Kolben mit 200 ml steriler Nährlösung entsprechender Zusammensetzung. Nach 30-stündiger Bebrütungszeit bei 29°C werden mit je 20 Kolben (4 l) 16 l Nährlösung obiger Zusammensetzung in einem 30-l-Kleinfermenter beimpft und bei 29°C mit 20 l Luft/min. und einer Rührgeschwindigkeit von 350 Upm fermentiert. Nach Erreichen einer bestimmten Keimzahl, z.B. 4 x 10$^9$ Keimen/ml, wird mit einer stündlichen Verdünnungsrate von 0,14 h$^{-1}$ eine 100%ige Umsetzung erreicht.

b) Impfgut und Vorkultur vom Stamm Serratia plymuthica werden entsprechend Beispiel 2a) hergestellt, jedoch mit einer Nährlösung von 98%iger Reinheit. Bei gleichen Fermentationsbedingungen wie unter 2a) aufgeführt, wird mit einer stündlichen Verdünnungsrate von 0,09 h$^{-1}$ eine 100%ige Umsetzung erreicht.

### Patentansprüche

1. Verfahren zur kontinuierlichen Isomerisierung von Saccharose zu Isomaltulose mit Hilfe von Mikroorganismen, dadurch gekennzeichnet, daß man isomaltulosebildende Mikroorganismen in einem saccharosehaltige Pflanzensäfte enthaltenden Nährmedium in an sich bekannter Weise bebrütet, nach Erreichen einer ausreichenden Zelldichte kontinuierlich weiterzüchtet, indem man mit einer Verdünnungsrate, die zwischen 0,05 und 0,3 pro Stunde liegt, kontinuierlich saccharosehaltige Lösungen zusetzt, nach Isomerisierung der Saccharose die Lösung von der Mikroorganismenmasse abtrennt und in an sich bekannter Weise reinigt und/oder zur Kristallisation bringt.

2. Verfahren nach Anspruch 1, dadurch gekennzeichnet, daß man dieses unter Züchtung von Protaminobacter rubrum Z 12 (CBS 574.77) durchführt.

### Claims

1. A process for the continuous isomerisation of sucrose into isomaltulose with the aid of micro-organisms, characterised in that isomaltulose-forming micro-organisms are cultured in a manner known per se in a culture medium having a content of sucrose-containing vegetable juices; after an adequate cell density has been achieved, culturing is continued in a continuous manner by adding sucrose-containing solutions continuously at a dilution rate between 0.05 and 0.3 per hour; after the sucrose has been isomerised the solution is separated from the micro-organism mass and purified in a manner known per se and/or made to crystallize.

2. A process according to claim 1, characterised in that it is conducted by culturing Protaminobacter rubrum Z 12 (CBS 574.77).

### Revendications

1. Procédé d'isomérisation continue de saccharose en isomaltulose au moyen de micro-organismes, caractérisé en ce qu'il consiste à faire incuber d'une manière connue des micro-organismes producteurs d'isomaltulose dans un milieu nutritif contenant des sucs végétaux chargés de saccharose, à poursuivre la culture continue après qu'une densité cellulaire suffisante a été atteinte, en ajoutant continuellement des solutions chargées de saccharose avec un taux de dilution qui se situe entre 0,05 et 0,3/h, à séparer après l'isomérisation du saccharose la solution de la masse de micro-organismes et à la purifier et/ou à la faire cristalliser d'une manière connue.

2. Procédé suivant la revendication 1, caractérisé en ce qu'il est mis en oeuvre par culture de Protaminobacter rubrum Z 12 (CBS 574.77).